# Europäisches Patentamt

# European Patent Office

# Office européen des brevets

(19)

(11) Veröffentlichungsnummer : **0 443 391 B1**

(12) **EUROPÄISCHE PATENTSCHRIFT**

(45) Veröffentlichungstag der Patentschrift :
04.05.94 Patentblatt 94/18

(51) Int. Cl.⁵ : **C07D 307/08**

(21) Anmeldenummer : **91101832.3**

(22) Anmeldetag : **09.02.91**

(54) **Verfahren zur Gewinnung von Tetrahydrofuran aus Gemischen, die Tetrahydrofuran, 1,4-Butandiol, gamma-Butyrolacton und Bernsteinsäureester enthalten.**

(30) Priorität : **20.02.90 DE 4005295**

(43) Veröffentlichungstag der Anmeldung :
**28.08.91 Patentblatt 91/35**

(45) Bekanntmachung des Hinweises auf die
Patenterteilung :
**04.05.94 Patentblatt 94/18**

(84) Benannte Vertragsstaaten :
**AT BE CH DE ES FR GB IT LI NL SE**

(56) Entgegenhaltungen :
EP-A- 0 304 696
DE-A- 2 710 265
DE-A- 2 834 038
GB-A- 1 487 200
CHEMICAL ABSTRACTS, Band 104, Nr. 16, 21.
April 1986, Columbus, Ohio, USA IIO,AKIRA et
al. "Tetrahydrofuran" Seite 117, Spalte 1, Zu-
sammenfassung-Nr. 131959h

(56) Entgegenhaltungen :
CHEMICAL ABSTRACTS, Band 105, Nr. 15, 13.
Oktober 1986, Columbus, Ohio, USAKOTKAR,
DILIP et al. "Cyclodehydration of nonaro- matic diols using aluminum(III)-exchanged mont-
morillo- nite as a solid Broensted acid
catalyst" Seite594, Spalte 1, Zusammenfas-
sung-Nr. 133 007r
CHEMICAL ABSTRACTS, Band 110, Nr. 1, 2.
JUnner 1989, Columbus, Ohio, USAKOTKAR,
DILIP et al. "Cyclodehydration of non- aromatic diols on Al(III)-montmorillonite clay. Reactivity and mechanism" Seite 731, Spalte 1, Zu-
sammenfassung-Nr. 7 978y

(73) Patentinhaber : **BASF Aktiengesellschaft
Carl-Bosch-Strasse 38
D-67063 Ludwigshafen (DE)**

(72) Erfinder : **Stabel, Uwe, Dr.
Buchenweg 2
W-6803 Edingen-Neckarhausen (DE)**
Erfinder : **Gosch, Hans-Juergen, Dr.
Seebacher Strasse 37
W-6702 Bad-Duerkheim (DE)**
Erfinder : **Fischer, Rolf, Dr.
Bergstrasse 98
W-6900 Heidelberg (DE)**
Erfinder : **Harder, Wolfgang, Dr.
Bergwaldstrasse 16
W-6940 Weinheim (DE)**
Erfinder : **Hechler, Claus, Dr.
Uhlandstrasse 37A
W-6800 Mannheim 1 (DE)**

EP 0 443 391 B1

## Beschreibung

Diese Erfindung betrifft ein Verfahren zur Gewinnung von Tetrahydrofuran aus Gemischen, die 1,4-Butandiol, Tetrahydrofuran, $\gamma$-Butyrolacton und Bernsteinsäureester enthalten und die bei der katalytischen Hydrierung von Maleinsäure, Bernsteinsäure, Fumarsäure, den Estern dieser Säuren, Maleinsäureanhydrid oder Bernsteinsäureanhydrid in Gegenwart von Alkoholen anfallen.

Im weiteren Text werden die folgenden Kurzbezeichnungen verwendet: MS = Maleinsäure, FS = Fumarsäure, BS = Bernsteinsäure, MSA = Maleinsäureanhydrid, BSA = Bernsteinsäureanhydrid, THF = Tetrahydrofuran, GBL = $\gamma$-Butyrolacton, BD = 1,4-Butandiol, BuOH = n-Butanol, BSDE = Bernsteinsäuredibutylester, POH = Propanol und HS = Hochsieder.

Es sind schon zahlreiche Verfahren zur katalytischen Hydrierung von MSA vorgeschlagen worden. Da bei der katalytischen Hydrierung von MSA, wie sie z.B. in der EP-A-304 696 beschrieben ist, wertvolle Zwischenprodukte, wie THF, GBL und BD gebildet werden, hat die MSA-Hydrierung großes technisches Interesse gefunden. Will man die MSA-Hydrierung mit dem Ziel einer möglichst hohen Ausbeute an THF durchführen, so gelingt dies durch die Reaktionsführung nur zum Teil, so daß man darauf angewiesen ist, das im Gemisch enthaltene BD nach Abtrennung zur Erhöhung der Gesamtausbeute an THF zu zyklisieren. Für die Zyklisierung zum THF wird BD bekanntlich mit sauren Katalysatoren, wie Schwefelsäure, Phosphorsäure, sauren Metalloxiden, wie $Al_2O_3$ und MgO, Zeolithen und sauren Schichtsilikaten umgesetzt. So ist z.B. aus J. Chem. Soc. Perkin Trans I, 1988, 1749-1751 bekannt, daß man aus BD durch einstündiges Erhitzen in Gegenwart von Montmorillonit auf 160°C THF in 93 %iger Ausbeute erhält.

Geht man bei der Zyklisierung von BD jedoch z.B. von einem Gemisch aus, das BD, GBL und Bernsteinsäureester enthält, so gelingt es nicht, BD ohne Verlust an GBL und Bernsteinsäureester vollständig zu THF umzusetzen. Zur Vermeidung dieser Schwierigkeiten müßte der Katalysator eine für die Zyklisierung in einer verdünnten Lösung sehr hohe Zyklisierungsaktivität besitzen, um vollständige Umsätze bei geringen Katalysatormengen zu erreichen. Andererseits dürfte das BD unter dem Einfluß der sauren Katalysatoren weder mit GBL noch mit Bernsteinsäureestern in nennenswertem Maße reagieren, um einen Verlust an Wertprodukt zu vermeiden. So werden bei der Zyklisierung der oben genannten Gemische mit Schwefelsäure als Katalysator bei 180°C beträchtliche Mengen BD, GBL und Bernsteinsäurederivate zu einer hochsiedenden Oligomerenmischung umgesetzt, die bei niederen Temperaturen zu einer wachsartigen Masse erstarrt. Das gleiche Ergebnis wird auch bei der Zyklisierung mit Aluminiumoxid als Katalysator beobachtet. Bei der Zyklisierung mit Magnesiumoxid beobachtet man eine Umesterung von Bernsteinsäureestern mit BD, wodurch die THF-Ausbeute beträchtlich reduziert wird.

Es war deshalb nach einem verfahren zu suchen, das es gestattet, die Zyklisierung von BD in Gemischen aus BD, GBL und Bernsteinsäureestern zum Zwecke eines möglichst vollständigen Umsatzes zu THF und unter Vermeidung von Verlusten der Ausbeuten an GBL und Bernsteinsäureestern durchzuführen.

Ferner sollte der Katalysator nach der Zyklisierung auf einfache Weise vollständig von den Wertprodukten abgetrennt werden können.

Es wurde nun gefunden, daß man THF aus Gemischen, die bei der katalytischen Hydrierung von MS, BS, FS, den Estern dieser Säuren, MSA oder BSA in Gegenwart von Alkoholen anfallen und die THF, BD, GBL, Bernsteinsäureester und den eingesetzten Alkohol enthalten, in der gewünschten vorteilhaften Weise gewinnen kann, wenn man aus den Gemischen THF, die Alkohole und Wasser abdestilliert, die Gemische dann in Gegenwart von Schichtsilikaten auf 100 bis 250°C erhitzt, das dabei sich bildende Tetrahydrofuran und Reaktionswasser aus dem Reaktionsgemisch abdestilliert und das Tetrahydrofuran aus dem Destillat abtrennt.

Überraschenderweise erzielt man mit dem erfindungsgemäßen Verfahren eine vollständige Umsetzung des im Gemisch vorhandenen BD unter milden Bedingungen mit einer Selektivität um 100 %. Die nach beendeter Zyklisierung erhaltene Wertproduktlösung aus GBL und Bernsteinsäureestern ist im Gegensatz zur bei der Zyklisierung mit Schwefelsäure oder Aluminiumoxid als Katalysator erhaltenen Produktlösung dünnflüssig. Eine nennenswerter Verlust an GBL und Bernsteinsäureester wird nicht beobachtet. Vorteilhafterweise ist der Katalysator aus der auch bei Raumtemperatur dünnflüssigen Lösung durch Filtration oder einstufige Verdampfung der Wertprodukte leicht abtrennbar. Gewünschtenfalls kann der Katalysator wiederverwendet werden.

Als Ausgangsgemische werden solche Gemische eingesetzt, die bei der katalytischen Hydrierung von MS, BS, FS, den Estern dieser Säuren, MSA oder BSA in Gegenwart von Alkoholen, wie Methanol, Ethanol, Propanol, Isopropanol oder in Gegenwart der verschiedenen, isomeren Butanole, wobei die Verwendung dieser Butanole, insbesondere die von n-Butanol, bevorzugt ist, anfallen und die THF, BD, GBL, Bernsteinsäureester, wie BSDE und die eingesetzten Alkohole enthalten. Außerdem können die Gemische BuOH, $H_2O$ und geringe Mengen an POH und HS enthalten. So werden als Ausgangsgemische z.B. die bei der in der EP-A-304 696 beschriebenen Hydrierung von MSA anfallenden Reaktionsgemische verwendet. Sie haben z.B. die folgende Zusammensetzung;

2

```
THF    0,1 bis 15, vorzugsweise  1   bis  7 Gew.-%
H₂O    3   bis 15, vorzugsweise  7   bis  9 Gew.-%
POH    0,1 bis  5, vorzugsweise  0,5 bis  1 Gew.-%
BuOH   20  bis 80, vorzugsweise  50  bis 60 Gew.-%
BSDE   1   bis 20, vorzugsweise  3   bis  8 Gew.-%
BD     10  bis 30, vorzugsweise  19  bis 21 Gew.-%
GBL    0,5 bis 10, vorzugsweise  1   bis  5 Gew.-%
```

Die Zyklisierung des BD in den Ausgangsgemischen nimmt man bei Temperaturen von 100°C bis 250°C, vorzugsweise bei 150°C bis 190°C, in Gegenwart der Schichtsilikate vor. Schichtsilikate sind schichtförmig aufgebaute Silikate, wie Aluminium- oder Magnesiumsilikate vom Typ der Montmorillonite, Bentonite oder Bleicherden. Besonders gut geeignet sind Schichtsilikate, beispielsweise Montmorillonite und Bentonite, die mit Wasserstoffionen oder mit Elementen der dritten Hauptgruppe des Periodensystems, insbesondere mit Bor, Aluminium, Gallium und/oder Indium dotiert sind, wobei die Verwendung Aluminium-dotierter Montmorillonite besonders bevorzugt wird. Die Schichtsilikate werden in Mengen von 0,05 bis 1 Gew.-% bezogen auf das BD eingesetzt.

Geht man von den obengenannten THF-enthaltenden Gemischen aus, die bei der katalytischen Hydrierung von MSA entstehen, so empfiehlt es sich, diese von den aus dem Hydrierkatalysator ausgewaschenen Spuren von Katalysatorbestandteilen zu befreien. Zu diesem Zweck wird der Hydrieraustrag vor der Zyklisierung bei Temperaturen bis 50°C mit einem handelsüblichen kontinuierlich betriebenen Kationenaustauscher entionisiert. Eine einstufige Verdampfung und Kondensation der Hydrierausträge erfüllt den gleichen Zweck. Man erreicht dadurch, daß die Zyklisierung mit geringen Katalysatormengen durchgeführt werden kann. Vor der Behandlung mit dem Schichtsilikat werden die Leichtsieder, wie THF, die Alkohole, z.B. das BuOH und Wasser abdestilliert. Man verwendet hierfür z.B. eine kontinuierlich betriebene Destillationskolonne, aus der die Leichtsieder z.B. bei einem Druck von 0,1 bis 2 bar über Kopf abdestilliert werden. Der Sumpfaustrag, der GBL, BSDE, BD und HS enthält, wird z.B. diskontinuierlich oder kontinuierlich in einem Rührkessel zur Zyklisierung des BD mit dem Katalysator umgesetzt. Das gebildete THF wird mit dem Reaktionswasser laufend abdestilliert und zweckmäßigerweise mit dem Leichtsiederdestillat vereinigt. Die Abtrennung des reinen THF erfolgt nach an sich bekannten Methoden, z.B. durch fraktionierte Destillation. Die Katalysatoren werden nach vollzogener Umsetzung des BD durch Filtration oder einstufige Verdampfung abgetrennt. Das verbliebene Gemisch aus GBL und BSDE kann in die Hydrierung des MSA zurückgeführt werden. Durch vollständige Rückführung der Wertprodukte in die Hydrierstufe entsteht aus einem Verfahren zur Herstellung von BD ein Verfahren, bei dem man THF mit hoher Selektivität und Gesamtausbeute erhält. Das Verfahren ermöglicht eine besonders wirtschaftliche THF-Gewinnung aus den genannten Reaktionsgemischen unter Vermeidung einer BD-Aufarbeitung.

Beispiel 1

Zyklisierung im diskontinuierlichen Rührkessel

Bei einer nach Beispiel 8 der EP-A-304 696 durchgeführten Hydrierung von MSA wurde ein Reaktionsgemisch mit der folgenden Zusammensetzung erhalten:

| | |
|---|---|
| THF | 6,4 Gew.-% |
| H₂O | 8,6 Gew.-% |
| POH | 0,5 Gew.-% |
| BuOH | 58,3 Gew.-% |
| BSDE | 4,2 Gew.-% |
| BD | 14,0 Gew.-% |
| GBL | 1,1 Gew.-% |
| Nebenprodukte | 6,9 Gew.-% |

1 bis 3 l/h des Gemisches wurden zur kontinuierlichen Entfernung von Kationen durch eine Glassäule (Durchmesser 50 mm, Länge 600 mm) gepumpt, die mit 1 l des im Handel unter der Bezeichnung Amberlyst® 15 erhältlichen Kationenaustauschers gefüllt war. Die Säule wurde bei Temperaturen von 20 bis 60°C betrieben. Um ein Ausbluten von Schwefelbestandteilen des Ionenaustauschers während der Entionisierung zu vermeiden, wurde der Ionenaustauscher vor Inbetriebnahme 24 h mit n-Butanol bei 60°C behandelt.

Zur kontinuierlichen Abtrennung der Leichtsieder wurde das entionisierte Gemisch in eine Kolonne gepumpt, deren Sumpf bei kontinuierlicher Reaktionsführung der Zyklisierung gleichzeitig als Reaktor genutzt wurde (Dosierung des Katalysators in den Sumpf).

Bei diskontinuierlicher Reaktionsführung wurde der leichtsiederfreie Sumpfaustrag in einen diskontinuierlichen Rührkessel mit aufgesetzter Destillationsbrücke überführt, mit 0,4 Gew.-% des Katalysators, bezogen auf das darin enthaltene BD, versetzt und zyklisiert.

Der Abtriebsteil der Kolonne zur Abtrennung der Leichtsieder bestand aus einer etwa auf Reaktionstemperatur beheizten Glockenbodenkolonne mit 5 Böden, um die Rückvermischung der hochsiedenen Bestandteile mit Leichtsiedern gering zu halten.

Der Verstärkungsteil bestand aus einer mit einem Drahtgestrickeinbau der Firma Sulzer (Sulzer-Packung) versehenen Kolonne (Breite 50 mm, Länge 400 mm). Die Kolonne wurde mit einem Rücklaufverhältnis von 0,5 bis 1,0 betrieben (Sumpftemperatur 170-190°C, Atmosphärendruck).

Nach destillativer Entfernung der Leichtsieder wurden dem Sumpfprodukt, zu dessen Zyklisierung 0,4 Gew.-% bezogen auf das darin enthaltene BD, eines mit Aluminium dotierten Montmorillonits zugegeben und diese Mischung auf 180°C erhitzt. Das sich hierbei bildende THF und das Reaktionswasser wurden kontinuierlich abdestilliert. Die Reaktion war nach 1.5 h beendet (Nachlassen des Siedens). Der nach dieser Umsetzung erhaltene Sumpfaustrag war auch bei Raumtemperatur dünnflüssig und enthielt als Hauptbestandteile GBL und BSDE. Das Destillat hatte folgende Zusammensetzung:

Wasser 10,0 Gew.-%
GBL 1,5 Gew.-%
BuOH 3,0 Gew.-%
THF 85,5 Gew.-%

Die Selektivität der Zyklisierung betrug 100 % bei 98 % Umsatz des Butandiols.

Der verwendete Katalysator wurde folgendermaßen hergestellt:

80 g Montmorillonit (pulverisiert) wurden in eine Lösung aus 480 g $Al(NO_3)_3 \cdot 9\ H_2O$ in 720 g $H_2O$ gegeben. Es wurde 4 h bei 80°C gerührt. Danach wurde der dotierte Montmorillonit abfiltriert, nitratfrei gewaschen und bei 100 bis 200°C im Trockenschrank getrocknet. Das in manchen Fällen zusammengebackene Pulver wurde im Mörser zerkleinert. Das im Überschuß angewandte Aluminiumnitrat kann für weitere Dotierungen wiederverwendet werden.

Beispiel 2

Zyklisierung im kontinuierlichen Rührkessel

Es wurden zwei nach dem in der EP-A-304 696 beschriebenen Verfahren erhältlichen Reaktionsgemische eingesetzt. Die Hydriergemische hatten die folgende Zusammensetzung:

Zusammensetzung der Hydriergemische (in Gew.-%):

|  | Hydriergemisch I vor der Zyklisierung | Produkte |
|---|---|---|
| THF | 6,4 | 18,3 |
| H₂O | 8,6 | 11,7 |
| POH | - | - |
| BuOH | 58,3 | 60,7 |
| BSDE | 4,2 | 3,7 |
| BD | 14,0 | 0,1 |
| GBL | 1,1 | 2,0 |
| Andere | 7,4 | 3,6 |
| Gesamtmasse | 8291 g | 8272 g |

Selektivität = 105 %    Umsatz BD = 99,1 %
Durchsatz = 1500 g/h

Die Selektivitätsangabe in Höhe von 105 % ist darauf zurückzuführen, daß das Hydriergemisch I BD-äquivalente Verbindungen, wie oligomere Ether des BD, enthielt, welche bei der Analyse des Hydriergemisches I nicht erfaßt worden waren und im Zuge der Zyklisierung in THF umgewandelt werden.

|  | Hydriergemisch II vor der Zyklisierung | Produkte |
|---|---|---|
| THF | 1,7 | 17,0 |
| $H_2O$ | 7,2 | 11,6 |
| POH | 0,2 | - |
| BuOH | 56,8 | 59,6 |
| BSDE | 6,7 | 6,7 |
| BD | 19,1 | 0,1 |
| GBL | 2,2 | 1,8 |
| Andere | 5,3 | 3,4 |
| Gesamtmasse | 5576 g | 5531 g |

Selektivität = 100 %    Umsatz BD = 99,6 %
Durchsatz = 1000 g/h

1000 bis 1500 g der jeweiligen Hydrieraustäge der Zusammensetzung I und II wurden wie in Beispiel 1 beschrieben entionisiert und die Leichtsieder abdestilliert. Das Sumpfprodukt wurde im Kolonnensumpf unter kontinuierlichem Zusatz von 0,2 bis 0,6 Gew.-% des in Beispiel 1 beschriebenen Katalysators, bezogen auf eingesetztes BD, bei 180 bis 190°C Reaktionstemperatur und einem Reaktionsvolumen von 0,7 bis 1,6 l zyklisiert. Das unter kräftigem Sieden gebildete THF und das Reaktionswasser wurden kontinuierlich aus dem Rührkessel abdestilliert und mit dem Leichtsiederdestillat vereinigt. Die mittlere Ausbeute an THF betrug im Mittel 99,9 % bezogen auf das in den Hydriergemischen vorhandene BD. Der kontinuierlich ausgetragene Reaktionssumpf war auch bei Raumtemperatur dünnflüssig und enthielt als Hauptbestandteile GBL und BSDE. Die Wertprodukte wurden nach einer Entfernung des Katalysators durch Filtration oder einstufige Verdampfung in die Hydrierung von MSA zurückgeführt.

Beispiele 3 bis 6

In den Beispielen 3 bis 6 wurde gemäß dem Verfahren von Beispiel 1, das dort gleichfalls eingesetzte, leichtsiederfreie, entionisierte Hydrierprodukt mit unterschiedlichen Katalysatoren und während unterschiedlicher Reaktionszeiten (RZ) zyklisiert. Als Zyklisierungskatalysatoren wurden γ-Aluminiumoxid (Vergleichsbeispiel), Tonsil® - ein säureaktivierter Calcium-Bentonit der Fa. Süd-Chemie - sowie ein säureaktivierter, d.h. ein mit Wasserstoffionen dotierter Montmorillonit verwendet. Die hinsichtlich der Selektivität (SEL), dem Umsatz und der Konsistenz des Zyklisierungsproduktes erzielten Ergebnisse sind in der folgenden Tabelle zusammen mit dem Ergebnis von Beispiel 1 aufgelistet:

| Beispiel Nr. | Katalysator | Reaktions- temperatur | SEL | UMSATZ | RZ | Konsistenz des Zyklisierungs- produkts nach der Reaktion |
|---|---|---|---|---|---|---|
| | | °C | % | % | h | |
| 3 | $\gamma$-Al$_2$O$_3$ | 238 | 57 | 68 | 5 | dickflüssig |
| 4 | Tonsil® | 180 | 97 | 97 | 4 | dünnflüssig |
| 5 | Tonsil® | 180 | 100 | 97 | 7 | dünnflüssig |
| 6 | Montmorillonit, H+- dotiert | 180 | 84 | 98 | 4 | dünnflüssig |
| 1 | Montmorillonit, Al$^{3+}$- dotiert | 180 | 100 | 98 | 1.5 | dünnflüssig |

**Patentansprüche**

1. Verfahren zur Gewinnung von Tetrahydrofuran aus Gemischen, die bei der katalytischen Hydrierung von Maleinsäure, Bernsteinsäure, Fumarsäure, den Estern dieser Säuren, Maleinsäureanhydrid oder Bernsteinsäureanhydrid in Gegenwart von Alkoholen anfallen und die Tetrahydrofuran, 1,4-Butandiol, $\gamma$-Buty- rolacton, Bernsteinsäureester und den eingesetzten Alkohol enthalten, dadurch gekennzeichnet, daß man aus den Gemischen Tetrahydrofuran, die Alkohole und Wasser abdestilliert, die Gemische dann in Gegenwart von Schichtsilikaten auf 100 bis 250°C erhitzt, das dabei sich bildende Tetrahydrofuran und Reaktionswasser aus dem Reaktionsgemisch abdestilliert und das Tetrahydrofuran aus dem Destillat abtrennt.

2. Verfahren nach Anspruch 1, dadurch gekennzeichnet, daß man Gemische einsetzt, die bei der katalytischen Hydrierung von Maleinsäureanhydrid in Gegenwart von Butanolen anfallen und die Tetrahydrofuran, Butanol, Propanol, 1,4-Butandiol, $\gamma$-Butyrolacton, Bernsteinsäuredibutylester und Wasser enthalten, wobei man aus dem Gemisch vor der Behandlung mit dem Schichtsilikat Tetrahydrofuran, Propanol, Butanol und Wasser abdestilliert.

3. Verfahren nach Anspruch 2, dadurch gekennzeichnet, daß man die Gemische vor der Behandlung mit dem Schichtsilikat von darin in Spuren enthaltenen Bestandteilen des Hydrierkatalysators befreit.

**Claims**

1. A process for isolating tetrahydrofuran from mixtures produced on catalytic hydrogenation of maleic acid, succinic acid, fumaric acid, the esters of these acids, maleic anhydride or succinic anhydride in the presence of alcohols and containing tetrahydrofuran, 1,4-butanediol, $\gamma$-butyrolactone, succinic acid esters and the alcohol employed, which comprises removing tetrahydrofuran, the alcohols and water from the mixtures by distillation, then heating the mixtures to from 100 to 250°C in the presence of phyllosilicates, removing the resultant tetrahydrofuran and water of reaction from the reaction mixture by distillation, and separating the tetrahydrofuran from the distillate.

2. A process as claimed in claim 1, wherein mixtures are employed which are produced on catalytic hydrogenation of maleic anhydride in the presence of butanols and contain tetrahydrofuran, butanol, propanol, 1,4-butanediol, $\gamma$-butyrolactone, dibutyl succinate and water, the treatment of the mixture with the phyllosilicate being preceded by removal of tetrahydrofuran, propanol, butanol and water by distillation.

3. A process as claimed in claim 2, wherein the mixtures are freed from hydrogenation catalyst constituents, present in traces, before treatment with the phyllosilicate.

**Revendications**

1. Procédé pour la récupération de tétrahydrofuranne à partir de mélanges qui sont formés au cours de l'hydrogénation catalytique d'acide maléique, d'acide succinique, d'acide fumarique, des esters de ces acides, d'anhydride maléique ou d'anhydride succinique en présence d'alcools, et qui contiennent du tétrahydrofuranne, du 1,4-butanediol, de la $\gamma$-butyrolactone, des esters d'acide succinique et l'alcool mis en réaction, caractérisé en ce qu'on chasse des mélanges, par distillation, du tétrahydrofuranne, les alcools et de l'eau, puis on chauffe les mélanges à 100-250°C en présence de phyllosilicates, on chasse du mélange réactionnel, par distillation, le tétrahydrofuranne et l'eau de réaction ainsi formés, et on sépare du distillat le tétrahydrofuranne.

2. Procédé selon la revendication 1, caractérisé en ce qu'on traite des mélanges qui sont formés au cours de l'hydrogénation catalytique d'anhydride maléique en présence de butanols et qui contiennent du tétrahydrofuranne, du butanol, du propanol, du 1,4-butanediol, de la $\gamma$-butyrolactone, du succinate de dibutyle et de l'eau, et en ce qu'avant le traitement par le phyllosilicate, on chasse du mélange, par distillation, du tétrahydrofuranne, du propanol, du butanol et de l'eau.

3. Procédé selon la revendication 2, caractérisé en ce qu'avant le traitement par le phyllosilicate, on débarrasse les mélanges des constituants du catalyseur d'hydrogénation qui y sont contenus à l'état de traces.